# EUROPEAN PATENT SPECIFICATION

(11) **EP 0 522 756 B1**
(45) Date of publication and mention of the grant of the patent: **31.01.1996**
(21) Application number: 92305922.4
(22) Date of filing: 26.06.1992
(51) Int. Cl.: A61K 7/48, A61K 7/04, C11D 3/37

(54) **Ampholyte terpolymers providing superior conditioning properties in skin and nail care products**
Ampholitische Terpolymere mit verbesserten Konditionierungseigenschaften in Shampoozusammensetzungen und Haarpflegemitteln
Terpolymères ampholyte avec des propriétés de conditionnement supérieur employer dans des shampooings et d'autres produits pour les soins des cheveux

(30) Priority: 28.06.1991 US 723003; 17.06.1992 US 896637
(43) Date of publication of application: 13.01.1993
(73) Proprietor: Calgon Corporation, Pittsburgh, Pennsylvania (US)
(72) Inventor: Shih-Ruey, Thomas, Pittsburgh PA 15237 (US); Matz, Gary F., Carnegie, PA 15106 (US); Vaughan, Craig W., Freedom, PA 150542 (US); Melby, Allen L., Zelienople, PA 16063 (US)
(74) Representative: Nash, David Allan

(56) References cited:
- EP-A- 0 266 111
- EP-A- 0 308 221
- WO-A-86/05391
- US-A- 4 401 650
- US-A- 4 528 111
- US-A- 4 806 345

## Description

### BACKGROUND OF THE INVENTION

### 1. Field of the Invention

The present invention relates to compositions and methods for treating the skin and nails in which a cosmetically acceptable medium is used which contains from 0.1-10% by weight of an ampholyte terpolymer.

The surface properties of human skin and nails are, of course, of basic interest in cosmetic science; and there has thus been a long-standing desire to discover ingredients which will beneficially affect the topical condition of this keratinous substrate. Skin conditioning products are desired which will function to improve such properties as retention of skin moisture, softening of the skin, attraction of air moisture, retardation of skin water loss, feel and reduction of skin irritations caused by contact with detergents, soaps and the like.

In this field, two broad areas of skin care products have been recognized as skin conditioners: emollients and humectants. Emollients function to provide improved moisture retention in the skin and plasticization/softening of the skin. Common commerical emollients are mineral oil; petrolatum; aliphatic alcohols, such as stearyl alcohol; lanolin and its derivatives; glycol stearate; and fatty acids, such as triethanolamine oleate. Humectants function to attract moisture, retard evaporation of water from the skin surface, and plasticize/soften skin. Common commercial humectants are glycerin, propylene glycol, sorbitols, and polyethylene glycols.

A desirable skin conditioner should impart all or some of the attributes of an emollient and a humectant, as well as provide improved lubricity and feel to the skin after treatment and/or reduce skin irritation caused by other components in the conditioner such as soaps, detergents, foam boosters, surfactants, perfumes and the like. Recently, cationic polymers have been used as skin conditioners.

Sometimes, it is also desirable that the ingredients of skin and nail care products will have adequate adherent properties, so that they are not only adsorbed initially, but are also retained on exposure to water. This property is referred to as "substantivity", i.e., the ability of a material to be adsorbed onto the keratin of the skin and nails and to resist removal by water rinse-off.

As indicated, human skin and nails are composed of keratin, a sulfur-containing fibrous protein. The isoelectric point of keratin, and thus of the hair of which it is composed, is in the pH range of 3.2-4. Thus, at the pH of typical use conditions, the human skin and nails carry a net negative charge. Consequently, cationic polymers have long been used as conditioners in nail and skin care formulations. The substantivity of the cationic polymers for negatively charged skin and nails leads to film formation that facilitates lubricity, moisturizing and feel. Two commercially used cationic polymers are Merquat 550 (Calgon), a copolymer of acrylamide and dimethyldiallylammonium chloride, and polymer JR (Union Carbide), a quaternary nitrogen-containing hydroxyethyl cellulose.

It is a feature of the ampholyte terpolymers of the present invention that by varying the molar amounts and/or types of cationic components of the overall terpolymer,it is possible to adjust the cationic nature of the amphoteric polymer. By altering the mole percent charge, distribution of the cationic charge on the polymer backbone, and distance from the backbone (side chain length), one can achieve variations in the lubricity, moisturizing and feel of the resulting ampholyte terpolymer.

When cationic polymers are added to skin care products which are cleaning composition, e.g., dishwashing liquids, that contain anionic surfactants, formation of highly surface active association complexes takes place which imparts improved foam stability to the product. Maximum surface activity and foam stability, or lather, are achieved at near stoichiometric ratios of anionic sufactant:cationic polymer, where the complex is least water soluble. All cationic conditioners exhibit some incompatibility at some of these ratios. While compatibility gives clear formulations, where that is desired, a certain amount of incompatibility is also acceptable.

The skin and nail conditioning properties of lubricity, moisturizing and feel are related to the film forming properties of the cationic monomers, as well as to molecular weight, with performance increasing with increasing molecular weight.

The ampholyte terpolymers of the present invention give one the ability, by varying the proportions and individual character of the nonionic, cationic and anionic components, to balance the hydrophobic and hydrophilic characteristics of the overall ampholyte terpolymer, thereby permitting one to optimize the conditioning properties of the final product.

It is also possible, by varying the types and proportions of the nonionic, cationic and anionic components, especially the anionic components, of the ampholyte terpolymers of the present invention, to be able to adjust the acidic nature, e.g., the pKₐ, of the anionic component of those terpolymers so as to more closely adjust the intrapolymer and polymer/skin and nail interactions, and thereby favorably affect the resulting conditioning properties.

While all of the conditioning property improvements described above are applicable primarily to skin care, human nail care products will also benefit from the improvement in conditioning properties afforded by the ampholyte terpolymers of the present invention. In addition, fragile or brittle nails will be strengthened or hardened, and the appearance of the nails will be improved as a result of use of the ampholyte terpolymers of the present invention.

Thus, overall the ampholyte terpolymers of the present invention are believed to constitute a significant improvement in the skin and nail conditioning art because, when contrasted to the products heretofore used in the art, they exhibit a superior combination of skin and nail care properties. These properties include improved moisture retention, and improved lubricity and feel of the skin and nails during and after treatment.

### 2. Brief Description of the Prior Art

Heretofore, skin and nail conditioning additives have been largely of three different types: cationic polymers, proteins or protein derivatives, and fatty quaternary ammonium compounds. Commonly used cationic polymers include: quaternary nitrogen-containing hydroxyethyl cellulose compounds, copolymers of vinylpyrrolidone and dimethylaminoethylmethacrylate, and amino functional polydimethylsiloxane. Hydrolyzed animal protein has been frequently used as a skin conditioner. Also used are natural products such as collagen and casein. Suitable quaternary ammonium compounds include such products as stearyl dimethyl ammonium chloride.

Conditioning additives comprising copolymers of dimethyldiallylammonium chloride and other monomers are well known; see, e.g., EP-A-0 308 189 (with acrylamide); and EP-A-0 308 190 (with hydroxyethyl cellulose). The use of such polymers in cosmetics is also described in Sykes et al., Drug Cosmet. Ind., 126(2), 62, 64, 66, 68, 136 (1980). Amphoteric betaines have also been employed in cosmetic compositions; see GB 2,113,245 which discloses use of betainized dialkylaminoalkyl(meth)acrylate together with a cationic polymer. EP-A-0 227 321 discloses copolymers of saccharides with cationic monomers used in making a mild soap bar.

While the use of various combinations of cationic, anionic and/or nonionic polymers as additives for hair, skin and nail conditioning compositions has been suggested heretofore, there has been no appreciation that a significant improvement in all of the desired conditioning properties could be obtained by employing an ampholyte terpolymer of the type used in the compositions and methods of the present invention.

For example, US 4,859,458 discloses hair conditioning polymers containing alkoxylated nitrogen salts of sulfonic acid which may also include additional monomers that may be neutral, anionic and/or cationic. While these include acrylamide, acrylic acid and dimethyldiallylammonium chloride, there is no suggestion of the ampholyte terpolymers of the present invention.

EP 0 353 987 discloses polymers for water-rinsable personal care products, comprising a cationic monomer including dimethyldiallylammonium chloride, a monomer that carries a pendant group AₙR where n is 0 or a positive integer, A is ethyleneoxy and R is a hydrocarbyl group of 8 to 30 carbon atoms, and optionally a nonionic and/or an anionic monomer. However, there is no suggestion of the ampholyte terpolymers of the present invention.

US 4,710,374 discloses cosmetic compositions comprising a cationic polymer including poly(dimethyldiallylammonium chloride), and an anionic latex, but there is no suggestion of the ampholyte terpolymers of the present invention.

US 4,842,849 discloses compositions suitable for treating the hair, skin and nails comprising at least one cationic polymer including poly(dimethyldiallylammonium chloride), and at least one anionic polymer containing vinylsulfonic groups, optionally copolymerized with acrylamide. The cationic polymer may be an amphoteric polymer as defined, but none of these combinations suggest the ampholyte terpolymers of the present invention.

EP 0 080 976 discloses aqueous hair-cosmetic compositions containing a surface active polymeric acrylic-based quaternary ammonium salt, a monomeric or oligomeric ammonium salt, and a surface active nonionic, anionic or zwitterionic component. The ampholyte terpolymers of the present invention are not suggested.

US 4,128,631 discloses a method of imparting lubricity to keratinous substrates such as skin or hair by contacting said substrates with a salt of 2-acrylamido-2-methylpropane sulfonic acid (AMPSA) having a molecular weight of from 1-5 million. See also US 4,065,422. The ampholyte terpolymers of the present invention and their unexpected advantageous properties are not suggested.

The ampholyte terpolymers of the present invention are regarded as novel compositions of matter because of their unique properties, and their use as skin and nail conditioning additives has not heretofore been suggested.

Terpolymers of acrylamide/dimethyldiallylammonium chloride/acrylic acid are disclosed in US 4,455,240; 4,460,477; 4,484,631; and 4,533,708; however, nowhere is there a suggestion that those terpolymers might be used as conditioning additives for hair products.

A graft copolymer of acrylamide/acrylic acid/amylopectin/dimethyldiallylammonium chloride is disclosed in US 4,131,576, but is only suggested for use as a pigment retention agent in papermaking.

The ampholyte terpolymers of the present invention represent a significant advance in the state of the skin and nail conditioning art and afford properties which are a surprising improvement over those possessed by the conditioning additives in the prior art described above. In contrast to such additives, the ampholyte terpolymers of the present invention provide either an optimized.combination of all conditioning properties, or else an optimized subset of such properties, which include: nail strengthening and appearance improvement, retention of skin moisture, softening of the skin, attraction of air moisture, retardation of skin water loss, feel and reduction of skin irritations caused by contact with detergents, soaps and the like.

### SUMMARY OF THE INVENTION

The present invention relates to a composition for treating human skin and nails in which a cosmetically acceptable medium is used which contains from 0.1-10% by weight of an ampholyte terpolymer having a weight average molecular weight of from about 10 thousand to 10 million, comprising (a) from at least 1 to as much as 95 weight percent of a nonionic monomer, (b) from at least 5 to as much as 80 weight percent of a cationic monomer, and (c) from at least 1 to as much as 75 weight percent of an anionic monomer.

In a preferred embodiment, the nonionic monomer component of the ampholyte terpolymer in the composition for treating human skin and nails is acrylamide or derivative thereof as defined hereafter; the cationic monomer component is a diallylamine derivative as defined hereafter; and the anionic derivative is acrylic acid or derivative thereof, as defined hereafter.

The present invention also relates to a method of treating human skin and nails which comprises applying to said skin and nails a cosmetically acceptable medium containing from 0.1-10% by weight of an ampholyte terpolymer having a weight average molecular weight of from about 10 thousand to 10 million, comprising (a) from at least 1 to as much as 95 weight percent of a nonionic monomer, (b) from at least 5 to as much as 80 weight percent of a cationic monomer, and (c) from at least 1 to as much as 75 weight percent of an anionic monomer.

In a preferred embodiment, the nonionic monomer component of the ampholyte terpolymer contained in the cosmetically acceptable medium used in treating human skin and nails is acrylamide or derivative thereof as defined hereafter; the cationic monomer component is a diallylamine derivative as defined hereafter; and the anionic derivative is acrylic acid or derivative thereof, as defined hereafter.

### DETAILED DESCRIPTION OF THE INVENTION

As already indicated, the present invention relates to a composition for treating human skin and nails in which a cosmetically acceptable medium is used which contains from 0.1-10% by weight of an ampholyte terpolymer having a weight average molecular weight of from about 10 thousand to 10 million, comprising (a) from at least 1 to as much as 95 weight percent of a nonionic monomer, (b) from at least 5 to as much as 80 weight percent of a cationic monomer, and (c) from at least 1 to as much as 75 weight percent of an anionic monomer.

### Components of the Ampholyte Terpolymers

Turning to each of the components of the ampholyte terpolymer in turn, the nonionic monomer comprises from 1 to 3 members independently selected from the group consisting of the following monomers and derivatives thereof:

| | |
|---|---|
| acrylamide (AM) | vinylacetate (VA) |
| N-alkylacrylamide (NAAM) | vinyl alcohol (VOH) |
| N-vinylpyrrolidinone (VP) | acrylate esters |
| methacrylamide (MAM) | allyl alcohol (AAlc) |

Derivatives of the above monomers are well known and those are useful in the present invention which provide the various conditioning properties described further herein. For example, N-alkylacrylamide and methacrylamide may be regarded as derivatives of acrylamide, and these together with other known derivatives of acrylamide are suitable and preferred for use in the ampholyte terpolymers of the present invention, and may be represented by the following general formula: 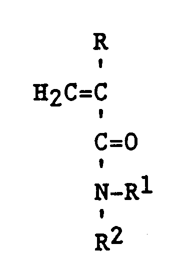
where R is H or CH₃; and R¹ and R² are independently H, C₁₋₄ alkyl, CH₂OCH₃, CH₂OCH₂CH(CH₃)₂, (CH₂CH₂O-)ₓ-H, where x=1-50, or phenyl, or together are C₃₋₆cycloalkyl.

The most preferred acrylamide monomer is the simplest, i.e., that where R, R₁, and R₂ are all H.

The nonionic monomer portion of the ampholyte terpolymers of the present invention is usually present in an amount of from 1 to 95 weight percent of the total terpolymer. Preferably, this amount is from 5 to 80 weight percent, and most preferably, this amount is from 10 to 50 weight percent. However, in the case of the monomers N-alkylacrylamide, vinyl acetate, vinyl alcohol, and acrylate esters, solubility problems are created when the proportion of these anionic monomers is at the higher end of the ranges recited above for the overall ampholyte terpolymer. Thus, the N-alkylacrylamide monomers should be present in amounts of from 1 to 10% by weight; and the vinyl acetate, vinyl alcohol, and acrylate esters monomers should be present in amounts of from 1 to 30% by weight as components of the overall ampholyte terpolymer.

As will be appreciated by the synthetic polymer chemist, the vinyl alcohol monomer will most conveniently be generated as a monomer simply by hydrolysis of vinyl acetate. Similarly, acrylic acid and methacrylic acid may be conveniently introduced into the terpolymer by hydrolysis of acrylamide and methacrylamide, respectively.

The acrylamide and other nonionic components of the ampholyte terpolymers of the present invention contribute to the film forming capacity of the total terpolymer and thus improve lubricity and feel of the overall terpolymer.

Where the amounts of cationic and anionic components are such that the net charge of the overall terpolymer is near zero, there is a tendency to form water insoluble polysalt complexes. The presence of acrylamide and the other nonionic monomer components serves to enhance the water solubility of the terpolymers in those situations.

It has also been discovered that, surprisingly, when the weight percent of acrylamide and other nonionic monomer components is reduced from about 50 to about 25 weight percent, while the balance of cationic and anionic components stays approximately the same, there is a substantial improvement in the conditioning properties of the terpolymer. Consequently, the most preferred range of weight percents for the acrylamide and other nonionic components is from 10 to 50.

The next component of the ampholyte terpolymers of the present invention is the cationic monomer, e.g., derivatives of diallylamine which are preferred, especially dimethyldiallylammonium chloride (DMDAAC), which is most preferred. More generally, the cationic monomer comprises 1 or 2 members independently selected from the group consisting of the following monomers and derivatives thereof:
dimethyldiallylammonium chloride (DMDAAC)
diallylamine (DAA)
methyldiallylamine (MDAA)
N,N-dialkyldiallylammonium chloride (R₁, R₂ higher than CH₃ in the formula below)
dimethylaminoethylmethacrylate (DMAEM)
methacyloyloxyethyl trimethylammonium chloride (METAC)
methacyloyloxyethyl trimethylammonium methyl sulfate (METAMS)
acryloyloxyethyl trimethylammonium chloride (AETAC)
dimethylaminopropylmethacrylamide (DMAPMA)
methacrylamidopropyl trimethylammonium chloride (MAPTAC)

Derivatives of the above cationic monomers are well known and those are useful in the present invention which provide the various conditioning properties described further herein. For example, dimethyldiallylammonium chloride, methyldiallylamine, and N,N-dialkyldiallylammonium chloride may be regarded as derivatives of diallylamine, and these together with other known derivatives of diallylamine are suitable and preferred for use in the ampholyte terpolymers of the present invention, and may be represented by the following general formula: 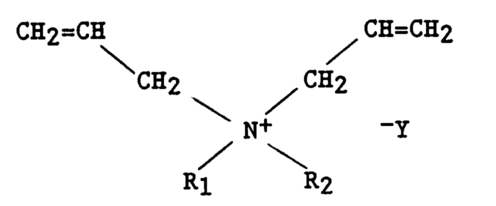
where R₁ and R₂ are independently H or C₁₋₁₂alkyl. The moiety ⁻Y is a suitable anion, such as halide, preferably ⁻Cl, but also including sulfate, and so forth. It is within the skill of the artisan to choose such an anion. The preferred cationic monomer component of the ampholyte terpolymers of the present invention is dimethyldiallylammonium chloride (DMDAAC), i.e., where R₁ and R₂ are CH₃.

The cationic monomer portion of the ampholyte terpolymers of the present invention is present in an amount of from 5 to 80 weight percent of the total terpolymer. Preferably, this amount is from 15 to 60 weight percent. However, in the case of the monomers N,N-dialkyldiallylammonium chloride, i.e., where R₁ and R₂ are alkyl higher than methyl in the formula above, solubility problems are created when the proportion of these cationic monomers is at the higher end of the ranges recited above for the overall ampholyte terpolymer. Thus, the N,N-dialkyldiallylammonium chloride monomers should be present in amounts of from 1 to 10% by weight as components of the overall ampholyte terpolymer.

The dimethyldiallylammonium chloride and other cationic monomers contribute to all of the skin and nail conditioning properties. As cationic monomers they possess the inherent substantivity necessary for the overall terpolymer to function. They also provide the basic improvement in moisturizing, lubricity, feel and nail strengthening.

The final component of the ampholyte terpolymers of the present invention is the anionic monomer, e.g., acrylic acid (AA). More generally, the anionic monomer comprises 1 or 2 members independently selected from the group consisting of the following monomers and derivatives thereof:
acrylic acid (AA)
methacrylic acid (MAA)
2-acrylamido-2-methylpropanesulfonic acid (AMPSA)
crotonic acid (CA)
sodium vinyl sulfonate (SVS)
acrylamidoglycolic acid (AGly)
2-acrylamido-2-methylbutanoic acid (AMBA)
2-acrylamido-2-methylpropanephosphonic acid (AMPPA)
sodium vinyl phosphonate (SVP)
allyl phosphonic acid (APA)

Derivatives of the above anionic monomers are well known and those are useful in the present invention which provide the various conditioning properties described further herein. For example, methacrylic acid may be regarded as a derivative of acrylic acid, and it together with other known deiivatives of acrylic acid are suitable and preferred for use in the ampholyte terpolymers of the present invention, and may be represented by the following general formula: 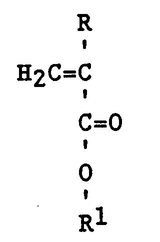
where R is H or CH₃; and R¹ is X⁺, H, C₁₋₄alkyl, CH₂CH₂OH, (CH₂CH₂O-)ₓ-H, where x=1-50, or phenyl, and X⁺ is a suitable cation forming a salt of the carboxylic acid, such as sodium, potassium, ammonium, monoethanolamine, etc., all of which are well known and within the ordinary skill of the artisan to choose.

The acrylic acid and other anionic monomer components of the ampholyte terpolymers of the present invention are present in an amount of from 1 to 75 weight percent of the total terpolymer. Preferably, this amount is from 5 to 40 weight percent. However, in the case of the monomers acrylamidoglycolic acid, solubility problems are created when the proportion of these anionic monomers is at the higher end of the ranges recited above for the overall ampholyte terpolymer. Thus, the acrylamidoglycolic acid monomers should be present in amounts of from 1 to 10% by weight as components of the overall ampholyte terpolymer.

The acrylic acid and other anionic monomers recited above, along with the acrylamide, contribute to the film forming capacity of the total terpolymer and thus improve moisturizing, lubricity, feel, and nail strengthening. In amounts as little as 1-2 weight percent, the acrylic acid and other anionic monomers measurably improve the compatibility of the overall terpolymer with the anionic surfactant of which a typical cleaning compositions are made. It has also been found that the acrylic acid and other anionic monomers add to the improvement of all of the skin and nail conditioning properties already provided by the cationic component as discussed above. This result is unexpected, and is another indication of the unexpected properties of the ampholyte terpolymers of the present invention.

It is possible to selectively optimize one or a subset of the desired conditioning properties described above, by proper proportioning of the nonionic, cationic and anionic components of the ampholyte perpolymers and their molecular weights. This may be a desired course of action responsive to an expression of particular consumer group preference for one or another of these various conditioning properties, depending on the makeup of that particular consumer group.

### Specific Ampholyte Terpolymers

Examples of specific preferred ampholyte terpolymers within the scope of the present invention are the following:

| | |
|---|---|
| AM/DMDAAC/AGly | acrylate esters/DMDAAC/AA |
| AM/DMDAAC/AMBA | AM/DMDAAC/AA/AMPSA/AMPPA |
| AM/DMDAAC/APA | acrylate esters/DMDAAC/AMBA |
| AM/DMDAAC/AMPPA | AM/DMDAAC/SVP |
| AAlc/DMDAAC/AA | |

Examples of specific more preferred ampholyte terpolymers within the scope of the present invention are the following:

| | |
|---|---|
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |

Examples of specific most preferred ampholyte terpolymers within the scope of the present invention are the following:

| | |
|---|---|
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

Within the group of most preferred terpolymers: AM/DMDAAC/AA, AM/DMDAAC/AMPSA, and AM/DMDAAC/AA/AMPSA, certain specific terpolymers having the following compositions with respect to the nonionic, cationic and anionic components, have been found to be particularly useful in the skin and nail conditioning compositions and methods of the present invention:

| Polymer Composition (Weight Percent) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |

| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

### Molecular Weights

The molecular weight of the ampholyte terpolymers of the present invention may be within the broad range of from about 10 thousand to about 10 million. The molecular weights of most of the specific terpolymers described herein are within the range of about 4 to 8 million. However, it is generally conceded that for polymers used in skin and nail conditioning, molecular weights over about 1 million add little to the effectiveness of those polymers in terms of conditioning properties. At the other end of the scale, polymers with relatively low molecular weights may also be effective in providing skin and nail conditioning properties. The ampholyte terpolymers of the present invention are also useful in weight average molecular weight ranges below 1 million, and even below 100 thousand, although generally the preferred molecular weight range will be from about 500 thousand to 5 million.

Reduced viscosity (dl/g) is used herein as an approximate measure of the weight average molecular weight of the ampholyte terpolymers of the present invention. Such values represent a capillary viscosity measured with a Ubbelohde Capillary Viscometer at 0.05% concentration of polymer in a 1M NaCl solution, pH 7, at 30°C. The resulting value is calculated in accordance with methods well known in the art.

### Preparation (Polymerization)

The ampholyte terpolymers of the present invention may be prepared in a straightforward manner by using the process described immediately below.

For each terpolymer composition the appropriate weights of aqueous nonionic component, e.g., acrylamide, and cationic component, e.g., DMDAAC, monomers are charged to a glass reactor equipped with stirring. The volume of anionic component, e.g., acrylic acid, monomer to be added is diluted first with deionized water and then added to the reactor with vigorous stirring to give a total monomer concentration of 14 - 20%. The monomer mixture is adjusted to pH 6.5 with dilute NaOH, heated to 55°C, and purged with nitrogen for at least thirty minutes. Polymerization is initiated by adding 5 x 10⁻² mole% of sodium persulfate and 2.4 x 10⁻³ mole% of sodium bisulfite. After the peak exotherm is reached, additional dilution water and sodium bisulfite are added to scavenge any residual monomer and to dilute the final product to 4 - 8% polymer solids.

As already noted further above, in the case of certain anionic components such as acrylic acid and methacrylic acid may be introduced into the terpolymer by adding the corresponding nonionic components acrylamide and methacrylamide, respectively, and then hydrolyzing these once they are a part of the terpolymer, or in the course of its preparation, to give the desired amount of corresponding anionic component in the terpolymer final product. It has also been mentioned that the nonionic component vinyl alcohol (VOH) is most conveniently prepared by hydrolysis of the corresponding vinyl acetate (VA), so that the latter is the component introduced during polymerization, to be hydroyzed later to the vinyl alcohol.

### Cosmetically Acceptable Media

The ampholyte terpolymers of the present invention are used as compositions for treating human skin and nails by incorporating them in a cosmetically acceptable medium in amounts of from 0.1-10% by weight of said terpolymer, and preferably in an amount of from 0.5 to 5% by weight of said terpolymer.

These compositions can be presented in various forms, i.e., various cosmetically acceptable media, such as a liquid, cream, emulsion, gel, thickening lotion or powder; they can contain water and also any cosmetically acceptable solvent, in particular monoalcohols, such as alkanols having 1 to 8 carbon atoms, like ethanol, isopropanol, benzyl alcohol and phenylethyl alcohol, polyalcohols, such as alkylene glycols, like glycerine, ethylene glycol and propylene glycol, and glycol ethers, such as mono-, di- and tri-ethylene glycol monoalkyl ethers, for example ethylene glycol monomethyl ether, ethylene glycol monomethyl ether and diethylene glycol monomethyl ether, used singly or in a mixture. These solvents can be present in proportions of up to as much as 70% by weight, relative to the weight of the total composition.

These compositions can also be packaged as an aerosol, in which case they can be applied either in the form of an aerosol spray or in the form of an aerosol foam.

As the propellant gas for these aerosols, it is possible to use, in particular, dimethyl ether, carbon dioxide, nitrogen, nitrous oxide and volatile hydrocarbons, such as butane, isobutane, propane and, possibly, chlorinated and fluorinated hydrocarbons, although the latter are falling into increasing environmental disfavor.

Preferred compositions can also contain electrolytes, such as aluminum chlorhydrate, alkali metal salts, e. g., sodium, potassium or lithium salts, these salts preferably being halides, such as the chloride or bromide, and the sulphate, or salts with organic acids, such as the acetates or lactates, and also alkaline earth metal salts, preferably the carbonates, silicates, nitrates, acetates, gluconates, pantothenates and lactates of calcium, magnesium and strontium.

These compositions can also be presented in the form of a powder or of lyophilisates to be diluted before use.

The compositions according to the present invention can contain any other ingredient normally used in cosmetics, such as perfumes, dyestuffs which can serve to color the composition itself or the skin and/or nails, preservatives, sequestering agents, thickeners, silicones, softeners, foam synergistic agents, foam stabilisers, sun filters, peptising agents and also anionic, non-ionic, cationic or amphoteric surface-active agents or mixtures thereof. A list of the surface-active agents which can be used according to the present invention is given in US 4,240,450; 4,445,521; and 4,719,099.

If the compositions are presented in the form of a thickened lotion or a gel, they contain thickeners in the presence or absence of a solvent. The thickeners which can be used are especially carbopol, xanthan gums, sodium alginates, gum arabic and cellulose derivatives, and it is also possible to achieve thickening by means of a mixture of polyethylene glycol stearate or distearate or by means of a mixture of a phosphoric acid ester and an amide. The concentration of thickener is suitably 0.05 to 15% by weight.

The pH of the dyeing compositions is generally 7 to 11 and can be adjusted to the desired value by adding an alkalizing agent.

As already mentioned, all alcohols and particularly the monohydric alcohols may be used as ingredients with the ampholyte terpolymers of the present invention. Alcohols (C₁-C₂₄) which are non-irritating to the skin such as methanol, ethanol, isopropanol, propyl, lauryl, myristyl, cetyl, and stearyl, as well as mixtures thereof, may be used. Polyols such as glycerine, or ethylene glycol or propylene glycol may be utilized advantageously with the ampholyte terpolymers of the present invention. The choice of the alcohol to be utilized with the particular terpolymer of the present invention will ordinarily be dictated by product aesthetics and the physical form of the composition. For instance, where liquid compositions are desired, the lower alcohols are preferably utilized, while solid or cream compositions within the scope of the present invention will normally require the higher alcohols. Where the skin and nail care formulations of the present invention contain ingredients other than the ampholyte terpolymer or the alcohol in substantial amounts, the choice of the particular alcohol becomes less important. For example, if the skin and nail care product is to be an abrasive hand cleaning product, then a large proportion of the product will be surface active agents and an abrasive, such as pumice or sand, thereby giving wide latitude to the choice of the particular alcohol.

A desirable variable of the present invention is the incorporation of water with the ampholyte terpolymer of the present invention. A resultant increase in viscosity of the water is noted with no adverse effects on the stability of the product. Thus, water is a highly suitable carrier which may be used as a vehicle for contacting the ampholyte terpolymer and the skin or nail substrate. The particular weight ratios at which the desirable increase in viscosity occurs for mixtures of the ampholyte terpolymer and water are respectively from about 1:10,000 to about 1:100. Preferably this ratio is in the range of from about 1:1000 to about 1:5000. Within the aforementioned range, highly viscous skin and nail care compositions are obtained with low solids content. Such compositions are desirable in that they allow compositions such as suntan or body lotions to be formulated in a thickened state, providing greater ease of application.

In the present invention the ampholyte terpolymer is ordinarily used at a level of about 0.001 gram per square centimeter to about 0.1 gram per square centimeter of the affected substrate.

The moisturizing, lubricating and other conditioning effects of the ampholyte terpolymers of the present invention may be obtained through using such diverse products as soap bars, dishwashing compositions, douches, hand and body lotions, suntan lotion, cold creams, preshave and after shave products, as well as cleansing or lotion pads and wound dressings, deodorant and antiperspirant products in stick, gel, lotion and aerosol forms, cosmetics including lipstick, rouge, mascara and eye liner, facial bases and powders, wrinkle and spot removing creams and lotions, and the like, and many other skin and nail care products. Listed below are materials which may be included in such skin and nail care products.

Hand and body lotions frequently contain emollients such as stearic acid, glycerol monostearate, mineral oil, glycerine, sesame oil, bees wax, lauryl, myristyl, cetyl or stearyl alcohols, lanolin, lecithin, sterols, isopropylmyristate, as well any other recognized emollients. Emollients are typically used in the compositions of the present invention at levels of from abut 1% to about 50% by weight.

Astringents and antiseptics may be incorporated into the compositions of the present invention. A preferred astringent material is zinc phenolsulfonate. The foregoing material exhibits not only astringent but also antiseptic qualities and is of particular use in preshave formulations to stiffen the beard. Humectants such as propylene glycol are also desirable ingredients for inclusion in skin and nail care products to prevent drying of the skin. Allantoin is included in such compositions for its soothing and healing effects upon injured skin.

The soap bar and dishwashing compositions of the present invention may contain all manner of anionic, nonionic, zwitterionic, amphoteric or cationic surfactants. Typically the surfactant will be present at from about 1% to about 70%, preferably about 3% to 35% by weight.

Most preferably, the dishwashing compositions of the present invention contain anionic surfactants which, for example, include alkylether sulfates, olefin sulfonates, alkyl and alkenyl sulfates, alkyl sulfonates, and alkylbenzene sulfonates. A particularly useful discovery is that the ampholyte terpolymer, when used with a surface active agent, enhances and prolongs suds life. Consumers using dishwashing products often tend to overuse the composition when the suds disappear from the surface of the dishpan. Thus, in the surfactant formulations of the present invention, the presence of the ampholyte terpolymer maintains the suds level, thus avoiding inadvertent overuse of the product by the consumer.

The soap bars of the present invention may either contain real soap, combinations of soap and synthetic surfactants, or may be formulated solely with synthetic surfactants, such as alkylbenzene sulfonates. For a more detailed disclosure of components which are ordinarily found either as surfactants or additives in dishwashing compositions, see US 3,963,649.

### EXAMPLES OF PREFERRED EMBODIMENTS

The following examples demonstrate skin conditioning improvement results which can be obtained with specific ampholyte terpolymers of the present invention, but are not intended to in any way limit the scope of the present invention.

### EXAMPLE 1

Each ampholyte terpolymer is evaluated sequentially for feel during use, feel during rinse off and feel after application. Performance is evaluated by independent observers on coded samples using ratings of 0 to 4 (poor to excellent).

Each ampholyte terpolymer (1 wt %) is blended with a commercial product and evaulated by feel. One hand of each observer is treated with 1 gram of commercial liquid soap and at the same time the other hand of each observer is treated with 1 gram of commercial product plus 1 wt % polymer. Simultaneously, the observers rate the feel of the products. For liquid soaps, the feel during wash, during rinse, and after treatment are evaluated. For hand lotions, both hands of each observer are treated with each formulation and compared to its previously evaluated untreated commercial hand lotion. The feel during application and after application is evaluated using again a rating of 0 to 4 (poor to excellent).

In addition to feel, moisture vapor transmission rates (MVTR) are determined; 3 mil films on laboratory paper toweling are prepared with the ampholyte terpolymers. Each film is attached to a glass container holding a desiccant, anhydrous CaCl₂, and placed in 92 to 96% RH at 86°F. The MVTR results indicate a high rate of water vapor transmission.

The percent pickup of air moisture is also determined for the ampholyte terpolymers. A 2.2 mil film of terpolymer on laboratory paper toweling will pick up a certain percentage of moisture at 86° F., 92 to 96% relative humidity in 24 hours. The laboratory paper, by itself, will have essentially no pickup of air moisture. The polymer film will have a soft, smooth feel both dry and after moisture pickup. This data indicates that the ampholyte terpolymers of the present invention function as humectants.

The following ampholyte terpolymers, when evaluated in accordance with the assays and tests described above, will be found to give good to excellent results:

| Polymer Composition (Weight Percent) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |

| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

The following examples illustrate various cosmetically acceptable media for preparing skin and nail care compositons using the ampholyte terpolymers of the present invention. In those Examples, the following terpolymer abbreviations are used:

### Polyampholyte A:

- By weight:: 25% Acrylamide
50% DMDAAC
25% Acrylic Acid

### Polyampholyte B:

- By weight:: 50% Acrylamide
25% DMDAAC
35% AMPSA

### Polyampholyte C:

- By weight:: 50% N-Vinylpyrrolidinone
40% DMDAAC
10% Acrylic Acid

### EXAMPLE 2

### After Shave

| WT. % | INGREDIENT |
|---|---|
| 47.5% | SDA 40 Alcohol |
| 3.0% | Propylene Glycol |
| 0.5% | Polyampholyte A |
| 48.0% | H₂O |
| 1.0% | Fragrance |

The addition of the polyampholyte to this formula will leave the skin with a soft silky feeling during application and a soft moist feeling after dry-down. Substantially, the same results will be obtained if the terpolymer concentration is varied between 0.1% and 5.0%.

### EXAMPLE 3

### Sunscreen

| WT. % | INGREDIENT |
|---|---|
| 5.00 | Octyl Dimethyl PABA |
| 5.00 | Octyl Methoxycinnamate |
| 4.00 | Glyceryl Stearate |
| 4.00 | Sorbitan Sesquioleate |
| 4.00 | Lanolin |
| 3.00 | C12-15 Alcohols Benzoate |
| 3.0 | Cocoa Butter |
| 0.5 | Jojoba Oil |
| 0.5 | Benzyl Alcohol |
| 0.5 | Dimethicone |
| 2.0 | Stearic Acid |
| 0.1 | Carbomer-941 |
| 62.3 | Water |
| 1.40 | Polyampholyte B |
| 0.10 | Disodium EDTA |
| 0.50 | Triethanolamine |
| 4.0 | Sorbitol |
| 0.1 | Tocopheryl Acetate |

Adding the polyampholyte to this formula will improve the feel of the product and provide a softer feeling to the skin after application.

### EXAMPLE 4

### Hand Lotion

| WT. % | INGREDIENT |
|---|---|
| 7.00 | Stearic Acid |
| 0.50 | C12-15 Alcohols Benzoate |
| 0.50 | Sorbitan Oleate |
| 2.50 | Polysorbate-60 |
| 10.0 | Sorbitol |
| 1.50 | Polyampholyte C |
| 78.00 | Water |

The polyampholyte will provide a softer, smooth feel to the dry skin. Similar results can be expected at polyampholyte concentrations of 0.2 to 10.0%.

### EXAMPLE 5

### Liquid Hand Soap

| WT. % | INGREDIENT |
|---|---|
| 65.09 | H₂O |
| 22.0 | Sodium C14-16 Olefin Sulfonate |
| 3.0 | Cocamidopropyl Betaine |
| 3.0 | Lauramide DEA |
| 0.53 | Chloroxylenol |
| 0.13 | Tetrasodium EDTA |
| 2.00 | Sodium Chloride |
| 0.25 | Citric Acid |
| 4.00 | Polyampholyte A |

The polyampholyte in this product will provide a richer feeling liquid soap and lather, and after rinsing, the skin will be noticeably softer and smoother. Similar properties will be obtained using polyampholyte concentrations of 0.1 to 10.0%.

### EXAMPLE 6

### Bath Oil Bar

| WT. % | INGREDIENT |
|---|---|
| 2.00 | Water |
| 10.00 | Propylene Glycol |
| 1.00 | Polyampholyte B |
| 69.00 | PPG-3 Myristyl Ether |
| 10.00 | Mineral Oil |
| 8.00 | Sodium Stearate |

The polyampholyte in this product will result in better skin feel after use. Similar results will be obtained using 0.1 to 10% polyampholyte.

### EXAMPLE 7

### Brushless Shaving Cream

| WT. % | INGREDIENT |
|---|---|
| 18.00 | Stearic Acid |
| 4.00 | Ethylene Glycol Monostearate |
| 4.00 | Lanolin Alcohol |
| 4.00 | C12-15 Alcohols Benzoate |
| 2.00 | Glycerin |
| 1.00 | Triethanolamine |
| 2.50 | Polyampholyte C |
| 64.50 | H₂O |

The polyampholyte will provide a creamier product with more slip and leave the skin feeling softer. Similar results will be obtained at polyampholyte concentrations of 0.3 to 7.5%.

## Claims

1. A composition for treating human skin and nails in which a cosmetically acceptable medium is used which contains from 0.1-10% by weight of a water-soluble ampholyte terpolymer having a weight average molecular weight of from about 10 thousand to 10 million, comprising:
(a) from at least 1 to as much as 95 weight percent of a nonionic monomer comprising from 1 to 3 members independently selected from the group consisting of the following monomers and derivatives thereof:
| | |
|---|---|
| acrylamide (AM) | vinylacetate (VA) |
| N-alkylacrylamide (NAAM) | vinyl alcohol (VOH) |
| N-vinylpyrrolidinone (VP) | acrylate esters |
| methacrylamide (MAM) | allyl alcohol (AAlc) |
(b) from at least 5 to as much as 80 weight percent of a cationic monomer comprising 1 or 2 members independently selected from the group consisting of the following monomers and derivatives thereof:
dimethyldiallylammonium chloride (DMDAAC)
diallylamine (DAA)
methyldiallylamine (MDAA)
N,N-dialkyldiallylammonium chloride
dimethylaminoethylmethacrylate (DMAEM)
methacyloyloxyethyl trimethylammonium chloride (METAC)
methacyloyloxyethyl trimethylammonium methyl sulfate (METAMS)
acryloyloxyethyl trimethylammonium chloride (AETAC)
dimethylaminopropylmethacrylamide (DMAPMA)
methacrylamidopropyl trimethylammonium chloride (MAPTAC)
and
(c) from at least 1 to as much as 75 weight percent of an anionic monomer comprising 1 or 2 members independently selected from the group consisting of the following monomers and derivatives thereof:
acrylic acid (AA)
methacrylic acid (MAA)
2-acrylamido-2-methylpropanesulfonic acid (AMPSA)
crotonic acid (CA)
sodium vinyl sulfonate (SVS)
acrylamidoglycolic acid (AGly)
2-acrylamido-2-methylbutanoic acid (AMBA)
2-acrylamido-2-methylpropanephosphonic acid (AMPPA)
sodium vinyl phosphonate (SVP)
allyl phosphonic acid (APA).

2. A composition according to Claim 1 wherein the weight percent of the nonionic component is from 10 to 80, the weight percent of the cationic component is from 15 to 60, and the weight percent of the anionic component is from 5 to 40.

3. A composition according to Claim 1 wherein the ampholyte terpolymer is a member selected from the group consisting of:
| | |
|---|---|
| AM/DMDAAC/AGly | acrylate esters/DMDAAC/AA |
| AM/DMDAAC/AMBA | AM/DMDAAC/AA/AMPSA/AMPPA |
| AM/DMDAAC/APA | acrylate esters/DMDAAC/AMBA |
| AM/DMDAAC/AMPPA | AM/DMDAAC/SVP |
| AAlc/DMDAAC/AA | |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

4. A composition according to Claim 3 wherein the ampholyte terpolymer is a member selected from the group consisting of
| Polymer Composition (Weight Percent) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |
| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

5. A composition according to Claim 1 wherein the cosmetically acceptable medium is a member selected from the group consisting of after shave, sunscreen, hand lotion, liquid hand soap, bath oil bar, brushless shaving cream, and dishwashing liquid.

6. A method of treating human skin and nails which comprises applying to said skin and nails a cosmetically acceptable medium containing from 0.1-10% by weight of a water-soluble ampholyte terpolymer having a weight average molecular weight of from about 10 thousand to 10 million, comprising:
(a) from at least 1 to as much as 95 weight percent of a nonionic monomer comprising from 1 to 3 members independently selected from the group consisting of the following monomers and derivatives thereof:
| | |
|---|---|
| acrylamide (AM) | vinylacetate (VA) |
| N-alkylacrylamide (NAAM) | vinyl alcohol (VOH) |
| N-vinylpyrrolidinone (VP) | acrylate esters |
| methacrylamide (MAM) | allyl alcohol (AAlc) |
(b) from at least 5 to as much as 80 weight percent of a cationic monomer comprising 1 or 2 members independently selected from the group consisting of the following monomers and derivatives thereof:
dimethyldiallylammonium chloride (DMDAAC)
diallylamine (DAA)
methyldiallylamine (MDAA)
N,N-dialkyldiallylammonium chloride
dimethylaminoethylmethacrylate (DMAEM)
methacyloyloxyethyl trimethylammonium chloride (METAC)
methacyloyloxyethyl trimethylammonium methyl sulfate (METAMS)
acryloyloxyethyl trimethylammonium chloride (AETAC)
dimethylaminopropylmethacrylamide (DMAPMA)
methacrylamidopropyl trimethylammonium chloride (MAPTAC)
and
(c) from at least 1 to as much as 75 weight percent of an anionic monomer comprising 1 or 2 members independently selected from the group consisting of the following monomers and derivatives thereof:
acrylic acid (AA)
methacrylic acid (MAA)
2-acrylamido-2-methylpropanesulfonic acid (AMPSA)
crotonic acid (CA)
sodium vinyl sulfonate (SVS)
acrylamidoglycolic acid (AGly)
2-acrylamido-2-methylbutanoic acid (AMBA)
2-acrylamido-2-methylpropanephosphonic acid (AMPPA)
sodium vinyl phosphonate (SVP)
allyl phosphonic acid (APA).

7. A method according to Claim 6 wherein the weight percent of the nonionic component is from 10 to 80, the weight percent of the cationic component is from 15 to 60, and the weight percent of the anionic component is from 5 to 40.

8. A method according to Claim 6 wherein the ampholyte terpolymer is a member selected from the group consisting of:
| | |
|---|---|
| AM/DMDAAC/AGly | acrylate esters/DMDAAC/AA |
| AM/DMDAAC/AMBA | AM/DMDAAC/AA/AMPSA/AMPPA |
| AM/DMDAAC/APA | acrylate esters/DMDAAC/AMBA |
| AM/DMDAAC/AMPPA | AM/DMDAAC/SVP |
| AAlc/DMDAAC/AA | |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

9. A method accroding to Claim 8 wherein the ampholyte terpolymer is a member selected from the group consisting of:
| Polymer Composition (Weight Percent) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |
| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

10. A method according to Claim 6 wherein the cosmetically acceptable medium is a member selected from the group consisting of after shave, sunscreen, hand lotion, liquid hand soap, bath oil bar, brushless shaving cream, and dishwashing liquid.

## Patentansprüche

1. Zusammensetzung zur Behandlung menschlicher Haut und Nägel, wobei ein kosmetisch verträgliches Medium verwendet wird, das 0,1 bis 10 Gew.% wasserlösliches Ampholyt-Terpolymer mit einer massegemittelten Molekülmasse von etwa 10 Tausend bis 10 Millionen enthält, umfassend:
(a) mindestens 1 bis zu 95 Gew.% nichtionisches Monomer, umfassend 1 bis 3 Stoffe, unabhängig voneinander ausgewählt aus der Gruppe mit folgenden Monomeren und ihren Derivaten:
| | |
|---|---|
| Acrylamid (AM) | Vinylacetat (VA) |
| N-Alkylacrylamid (NAAM) | Vinylalkohol (VOH) |
| N-Vinylpyrrolidinon (VP) | Acrylatester |
| Methacrylamid (MAM) | Allylalkohol (AAlc) |
(b) mindestens 5 bis zu 80 Gew.% kationisches Monomer, umfassend 1 oder 2 Stoffe, unabhängig voneinander ausgewählt aus der Gruppe mit folgenden Monomeren und ihren Derivaten:
Dimethyldiallylammoniumchlorid (DMDAAC)
Diallylamin (DAA)
Methyldiallylamin (MDAA)
N, N-Dialkyldiallylammoniumchlorid
Dimethylaminoethylmethacrylat (DMAEM)
Methacyloyloxyethyltrimethylammoniumchlorid (METAC)
Methacyloyloxyethyltrimethylammoniummethylsulfat (METAMS)
Acryloyloxyethyltrimethylammoniumchlorid (AETAC)
Dimethylaminopropylmethacrylamid (DMAPMA)
Methacrylamidopropyltrimethylammoniumchlorid (MAPTAC)
und
(c) mindestens 1 bis zu 75 Gew.% anionisches Monomer, umfassend 1 oder 2 Stoffe, unabhängig voneinander ausgewählt aus der Gruppe mit folgenden Monomeren und ihren Derivaten:
Acrylsäure (AA)
Methacrylsäure (MAA)
2-Acrylamido-2-methylpropansulfonsäure (AMPSA)
Crotonsäure (CA)
Natriumvinylsulfonat (SVS)
Acrylamidoglycolsäure (AGly)
2-Acrylamido-2-methylbuttersäure (AMBA)
2-Acrylamido-2-methylpropanphosphonsäure (AMPPA)
Natriumvinylphosphonat (SVP)
Allylphosphonsäure (APA).

2. Zusammensetzung nach Anspruch 1, wobei der nicht-ionische Bestandteil 10 bis 80 Gew.%, der kationische Bestandteil 15 bis 60 Gew.% und der anionische Bestandteil 5 bis 40 Gew.% ausmacht.

3. Zusammensetzung nach Anspruch 1, wobei das Ampholyt-Terpolymer ein Stoff ist, ausgewählt aus der Gruppe, mit:
| | |
|---|---|
| AM/DMDAAC/AGly | Acrylatester/DMDAAC/AA |
| AM/DMDAAC/AMBA | AM/DMDAAC/AA/AMPSA/AMPPA |
| AM/DMDAAC/APA | Acrylatester/DMDAAC/AMBA |
| AM/DMDAAC/AMPPA | AM/DMDAAC/SVP |
| AAlc/DMDAAC/AA | |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

4. Zusammensetzung nach Anspruch 3, wobei das Ampholyt-Terpolymer ein Stoff ist, ausgewählt aus der Gruppe, mit:
| Polymer-Zusammensetzung (Gew.%) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |
| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

5. Zusammensetzung nach Anspruch 1, wobei das kosmetisch verträgliche Medium ein Stoff ist, ausgewählt aus der Gruppe mit After Shave, Sonnenschutz, Handlotion, flüssige Handseife, Badeöl-Kern, pinselfreie Rasiercreme und Geschirrspülflüssigkeit.

6. Verfahren zur Behandlung menschlischer Haut und Nägel, umfassend die Anwendung auf Haut und Nägel eines kosmetisch verträglichen Mediums, das 0,1 bis 10 Gew.% wasserlösliches Ampholyt-Terpolymer mit einer massegemittelten Molekülmasse von etwa 10 Tausend bis 10 Millionen enthält, umfassend:
(a) mindestens 1 bis zu 95 Gew.% nichtionisches Monomer, umfassend 1 bis 3 Stoffe, unabhängig voneinander ausgewählt aus der Gruppe mit folgenden Monomeren und ihren Derivaten:
Acrylamid (AM) Vinylacetat (VA)
N-Alkylacrylamid (NAAM) Vinylalkohol (VOH)
N-Vinylpyrrolidinon (VP) Acrylatester
Methacrylamid (MAM) Allylalkohol (AAlc)
(b) mindestens 5 bis zu 80 Gew.% kationisches Monomer, umfassend 1 oder 2 Stoffe, unabhängig voneinander ausgewählt aus der Gruppe mit folgenden Monomeren und ihren Derivaten:
Dimethyldiallylammoniumchlorid (DMDAAC)
Diallylamin (DAA)
Methyldiallylamin (MDAA)
N,N-Dialkyldiallylammoniumchlorid
Dimethylaminoethylmethacrylat (DMAEM)
Methacyloyloxyethyltrimethylammoniumchlorid (METAC)
Methacyloyloxyethyltrimethylammoniummethylsulfat (METAMS)
Acryloyloxyethyltrimethylammoniumchlorid (AETAC)
Dimethylaminopropylmethacrylamid (DMAPMA)
Methacrylamidopropyltrimethylammoniumchlorid (MAPTAC)
und
(c) mindestens 1 bis 75 Gew.% anionisches Monomer, umfassend 1 oder 2 Stoffe, unabhängig voneinander ausgewählt aus der Gruppe mit folgenden Monomeren und ihren Derivaten:
Acrylsäure (AA)
Methacrylsäure (MAA)
2-Acrylamido-2-methylpropansulfonsäure (AMPSA)
Crotonsäure (CA)
Natriumvinylsulfonat (SVS)
Acrylamidoglycolsäure (AGly)
2-Acrylamido-2-methylbuttersäure (AMBA)
2-Acrylamido-2-methylpropanphosphonsäure (AMPPA)
Natriumvinylphosphonat (SVP)
Allylphosphonsäure (APA).

7. Verfahren nach Anspruch 6, wobei der nichtionische Bestandteil 10 bis 80 Gew.%, der kationische Bestandteil 15 bis 60 Gew.% und der anionische Bestandteil 5 bis 40 Gew.% ausmacht.

8. Verfahren nach Anspruch 6, wobei das Ampholyt-Terpolymer ein Element ist, ausgewählt aus der Gruppe mit:
| | |
|---|---|
| AM/DMDAAC/AGly | Acrylatester/DMDAAC/AA |
| AM/DMDAAC/AMBA | AM/DMDAAC/AA/AMPSA/AMPPA |
| AM/DMDAAC/APA | Acrylatester/DMDAAC/AMBA |
| AM/DMDAAC/AMPPA | AM/DMDAAC/SVP |
| AAlc/DMDAAC/AA | |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

9. Verfahren nach Anspruch 8, wobei das Ampholyt-Terpolymer ein Stoff ist, ausgewählt aus der Gruppe, mit:
| Polymer-Zusammensetzung (Gew.%) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |
| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

10. Verfahren nach Anspruch 6, wobei das kosmetisch verträgliche Medium ein Stoff ist, ausgewählt aus der Gruppe mit After Shave, Sonnenschutz, Handlotion, flüssige Handseife, Badeöl-Kern, pinselfreie Rasiercreme und Geschirrspülflüssigkeit.

## Revendications

1. Composition pour traiter la peau et les ongles humains, dans laquelle on utilise un milieu cosmétiquement acceptable qui contient de 0,1 à 10% en poids d'un terpolymère ampholyte hydrosoluble ayant une masse moléculaire moyenne en poids d'environ 10 mille à 10 millions, comprenant:
(a) d'au moins 1 jusqu'à 95 pourcent en poids d'un monomère non ionique comprenant de 1 à 3 éléments indépendamment choisis dans le groupe formé par les monomères suivants et leurs dérivés:
| | |
|---|---|
| Acrylamide (AM) | Acétate de vinyle (VA) |
| N-alkylacrylamide (NAAM) | Alcool vinylique (VOH) |
| N-vinylpyrrolidinone (VP) | Esters acrylate |
| Méthacrylamide (MAM) | Alcool allylique (AAlc) |
(b) d'au moins 5 jusqu'à 80 pourcent en poids d'un monomère cationique comprenant 1 ou 2 éléments indépendamment choisis dans le groupe formé par les monomères suivants et leurs dérivés:
le chlorure de diméthyldiallylammonium (DMDAAC),
la diallylamine (DAA),
la méthyldiallylamine (MDAA),
le chlorure de N,N-dialkyldiallylammonium,
le méthacrylate de diméthylaminoéthyle (DMAEM),
le chlorure de méthacryloyloxyéthyltriméthylammonium (METAC),
le méthylsulfate de méthacryloyloxyéthyltriméthylammonium (METAMS),
le chlorure d'acryloyloxyéthyltriméthylammonium (AETAC),
le diméthylaminopropylméthacrylamide (DMAPMA),
le chlorure de méthacrylamidopropyltriméthylammonium (MAPTAC).
et
(c) d'au moins 1 jusqu'à 75 pourcent en poids d'un monomère anionique comprenant 1 ou 2 éléments indépendamment choisis dans le groupe formé par les monomères suivants et leurs dérivés:
l'acide acrylique (AA)
l'acide méthacrylique (MAA)
l'acide 2-acrylamido-2-méthylpropanesulfonique (AMPSA)
l'acide crotonique (CA)
le vinylsulfonate de sodium (SVS)
l'acide acrylamidoglycolique (AGly)
l'acide 2-acrylamido-2-méthylbutanoïque (AMBA)
l'acide 2-acrylamido-2-méthylpropanephosphonique (AMPPA)
le vinylsulfonate de sodium (SVP)
l'acide allylphosphonique (APA).

2. Composition selon la revendication 1, dans laquelle le pourcentage en poids du composant non ionique est compris entre 10 et 80, le pourcentage en poids du composant cationique est compris entre 15 et 60, et le pourcentage en poids du composant anionique est compris entre 5 et 40.

3. Composition selon la revendication 1, dans laquelle le terpolymère ampholyte est un élément choisi dans le groupe formé par:
| | |
|---|---|
| AM/DMDAAC/AGly | esters acrylate/DMDAAC/AA |
| AM/DMDAAC/AMBA | AM/DMDAAC/AA/AMPSA/AMPPA |
| AM/DMDAAC/APA | esters acrylate/DMDAAC/AMBA |
| AM/DMDAAC/AMPPA | AM/DMDAAC/SVP |
| AAlc/DMDAAC/AA | |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

4. Composition selon la revendication 3, dans laquelle le terpolymère ampholyte est un élément choisi dans le groupe formé par:
| Composition polymère (pourcent en poids) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |
| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

5. Composition selon la revendication 1, dans laquelle le milieu cosmétiquement acceptable est un élément choisi dans le groupe formé par un après-rasage, une crème solaire, une lotion pour les mains, un savon liquide pour les mains, un pain d'huile de bain, une crème de rasage sans blaireau et un liquide de lavage de vaisselle.

6. Procédé pour traiter la peau et les ongles humains qui consiste à appliquer à ladite peau et auxdits ongles un milieu cosmétiquement acceptable contenant de 0,1 à 10% en poids d'un terpolymère ampholyte hydrosoluble ayant une masse moléculaire moyenne en poids comprise entre environ 10 mille et 10 millions, comprenant:
(a) d'au moins 1 jusqu'à 95 pourcent en poids d'un monomère non ionique comprenant de 1 à 3 éléments indépendamment choisis dans le groupe formé par les monomères suivants et leurs dérivés:
| | |
|---|---|
| Acrylamide (AM) | Acétate de vinyle (VA) |
| N-alkylacrylamide (NAAM) | Alcool vinylique (VOH) |
| N-vinylpyrrolidinone (VP) | Esters acrylate |
| Méthacrylamide (MAM) | Alcool allylique (AAlc) |
(b) d'au moins 5 jusqu'à 80 pourcent en poids d'un monomère cationique comprenant 1 ou 2 éléments indépendamment choisis dans le groupe formé par les monomères suivants et leurs dérivés:
le chlorure de diméthyldiallylammonium (DMDAAC),
la diallylamine (DAA),
la méthyldiallylamine (MDAA),
le chlorure de N,N-dialkyldiallylammonium,
le méthacrylate de diméthylaminoéthyle (DMAEM),
le chlorure de méthacryloyloxyéthyltriméthylammonium (METAC),
le méthylsulfate de méthacryloyloxyéthyltriméthylammonium (METAMS),
le chlorure d'acryloyloxyéthyltriméthylammonium (AETAC),
le diméthylaminopropylméthacrylamide (DMAPMA),
le chlorure de méthacrylamidopropyltriméthylammonium (MAPTAC).
et
(c) d'au moins 1 jusqu'à 75 pourcent en poids d'un monomère anionique comprenant 1 ou 2 éléments indépendamment choisis dans le groupe formé par les monomères suivants et leurs dérivés:
l'acide acrylique (AA)
l'acide méthacrylique (MAA)
l'acide 2-acrylamido-2-méthylpropanesulfonique (AMPSA)
l'acide crotonique (CA)
le vinylsulfonate de sodium (SVS)
l'acide acrylamidoglycolique (AGly)
l'acide 2-acrylamido-2-méthylbutanoïque (AMBA)
l'acide 2-acrylamido-2-méthylpropanephosphonique (AMPPA)
le vinylsulfonate de sodium (SVP)
l'acide allylphosphonique (APA).

7. Procédé selon la revendication 6, dans lequel le pourcentage en poids du composant non ionique est compris entre 10 et 80, le pourcentage en poids du composant cationique est compris entre 15 et 60, et le pourcentage en poids du composant anionique est compris entre 5 et 40.

8. Procédé selon la revendication 6, dans lequel le terpolymère ampholyte est un élément choisi dans le groupe formé par:
| | |
|---|---|
| AM/DMDAAC/AGly | esters acrylate/DMDAAC/AA |
| AM/DMDAAC/AMBA | AM/DMDAAC/AA/AMPSA/AMPPA |
| AM/DMDAAC/APA | esters acrylate/DMDAAC/AMBA |
| AM/DMDAAC/AMPPA | AM/DMDAAC/SVP |
| AAlc/DMDAAC/AA | |
| AM/MDAA/AA | VP/DAA/AA |
| VP/METAMS/MAA/AA | VP/DMDAAC/AMPSA |
| MAM/METAMS/MAA | AM/VP/DMDAAC/AMPSA |
| AM/VP/METAMS/MAA | AM/VP/DMDAAC/AA/AMPSA |
| AM/VP/METAMS/MAA/AA | AM/DMDAAC/AETAC/AA |
| VP/AETAC/AA | AM/DMDAAC/DMAEM/AA |
| AM/DMDAAC/AA | AM/DMDAAC/AMPSA |
| AM/DMDAAC/AA/AMPSA | AM/MAPTAC/AMPSA |
| AM/DMDAAC/MAA | AM/MAPTAC/AA |
| AM/DMDAAC/CA | VP/DMDAAC/AA |
| AM/DMDAAC/SVS | AM/VP/DMDAAC/AA |
| AM/DAA/AA | AM/MAM/DMDAAC/AA |
| AM/DAA/MAA | AM/NAAM/DMDAAC/AA |
| AM/DMAEM/AA | AM/DAA/AMPSA |
| AM/AETAC/AA | AM/VA/VOH/DMDAAC/AA |
| AM/METAMS/MAA | VP/METAMS/MAA |

9. Procédé selon la revendication 8, dans lequel le terpolymère ampholyte est un élément choisi dans le groupe formé par:
| Composition polymère (pourcent en poids) | | |
|---|---|---|
| AM | DMDAAC | AA |
| 50 | 20 | 30 |
| 60 | 20 | 20 |
| 77 | 16 | 7 |
| 25 | 50 | 25 |
| 25 | 5 | 70 |
| 75 | 5 | 20 |
| 50 | 5 | 45 |
| 50 | 40 | 10 |
| 50 | 35 | 15 |
| 50 | 30 | 20 |
| AM | DMCAAC | AMPSA |
|---|---|---|
| 50 | 22 | 28 |
| 50 | 30 | 10 + 10 AA |
| 50 | 15 | 35 |
| 50 | 40 | 10 |
| 60 | 30 | 10 |
| 49 | 37 | 14 |
| 50 | 40 | 10 |

10. Procédé selon la revendication 6, dans lequel le milieu cosmétiquement acceptable est un élément choisi dans le groupe formé par un après-rasage, une crème solaire, une lotion pour les mains, un savon liquide pour les mains, un pain d'huile de bain, une crème de rasage sans blaireau et un liquide de lavage de vaisselle.
